# EUROPÄISCHE PATENTSCHRIFT

(11) **EP 1 498 147 B1**
(45) Veröffentlichungstag und Bekanntmachung des Hinweises auf die Patenterteilung: **25.02.2015**
(21) Anmeldenummer: 03016148.3
(22) Anmeldetag: 16.07.2003
(51) Int. Cl.: A61L 27/18, A61L 31/06, A61L 17/10, A61L 17/12

(54) **Abbaubares biokompatibles Blockcopolymer**
Absorbable biocompatible block copolymer
Copolymère bloc résorbable et biocompatible

(43) Veröffentlichungstag der Anmeldung: 19.01.2005
(73) Patentinhaber: Eidgenössische Technische Hochschule Zürich, 8092 Zürich (CH)
(72) Erfinder: Neuenschwander, Peter, 5400 Baden (CH)
(74) Vertreter: Schaad, Balass, Menzl & Partner AG

(56) Entgegenhaltungen:
- EP-A- 0 295 055
- EP-A- 0 552 896
- EP-A- 0 696 605
- DE-A- 4 224 401
- US-A- 4 281 077

## Beschreibung

Die Erfindung betrifft ein biokompatibles Blockcopolymer, enthaltend das Polykondensationsprodukt eines Diols und einer weiteren Komponente ausgewählt aus der Gruppe von des gleichen Diol, einem α,ω-Dihydroxy-polyester oder einem α,ω-Dihydroxy-polyether. Ausserdem betrifft die Erfindung ein medizinisches Implantat, enthaltend das. Blockcopolymer, die Verwendung des Blockcopolymers zur Herstellung eines medizinischen Implantats, sowie ein Diol und das Verfahren zur Herstellung desselben. Soweit der Ausdruck Medizin verwendet wird, wird darunter sowohl Human- als auch Veterinärmedizin verstanden.

Die Zahl der in der Praxis eingesetzten biokompatiblen Polymere für medizinische Implantate ist überraschend klein. Insbesondere gibt es praktisch keine elastischen biokompatiblen Polymere. Dies ist, ausser auf das Problem der Verträglichkeit, einerseits auf die hohen technischen Anforderungen bezüglich mechanischer Festigkeit, Sterilisierbarkeit, biologischer Abbaubarkeit und andererseits auf die Vielzahl verschiedener administrativer Vorschriften in den einzelnen Ländern zurückzuführen. Gerade die biologische Abbaubarkeit eines solchen Polymers stellt immense Anforderungen, da die gewünschte Abbaubarkeitsrate von der Verwendung stark abhängig ist.

Aus EP 0 696 605 ist ein biokompatibles Blockcopolymer bekannt, das als medizinisches Implantat verwendet werden kann. Dieses Blockcopolymer weist eine kristalline und eine amorphe Komponente auf. Die Abbaubarkeit dieser Blockcopolymere ist jedoch nicht für alle Anwendungen rasch genug.

Aufgabe der vorliegenden Erfindung ist die Schaffung eines neuen Polymers mit schnellerer Abbaubarkeit und nicht wesentlich veränderten biologischen Eigenschaften.

Diese Aufgabe wird durch das Blockcopolymer nach Anspruch 1 gelöst. Bevorzugte Ausführungsformen der Erfindung sind in den Ansprüchen 2-18 und in der Beschreibung beschrieben.

Es wurde festgestellt, dass das biokompatible Blockcopolymer und das Diol eine ausgesprochen gute Biokompatibilität aufweisen. Ausserdem kann durch den Einbau der Glycolid- oder Diglycolid-Einheiten die hydrolyrische und biologische Abbaubarkeitsrate des erfindungsgemässen biokomplatiblen Blockcopolymers und des Diols gesteuert werden. Da das Diol aus α- und/oder β -Hydroxyalkanoaten aufgebaut ist, werden beim Abbau dessen toxikologisch unbedenkliche Metaboliten gebildet. Intermediär werden, vorwiegend aus dem kristallinen Anteil des Polymers stammend, feste Partikel gebildet, die relativ klein sind und mittels Phagozytose aus dem Körper eliminiert werden. Die Grösse der wasserunlöslichen Partikel wird durch den Einbau der Diglycolid- oder Glycolideinheiten auch in das kristallisierbare Segment reduziert, wodurch die Phagocytose der Partikel erleichtert und beschleunigt wird.

Durch den Einbau des Diols in die erfindungsgemässen Blockcopolymere ist es möglich die Abbaurate der kristallinen Komponente zu beeinflussen. Mithin ist es möglich, die Abbaubarkeit solcher Blockcopolymere über die kristalline Komponente alleine, die amorphe Komponente alleine oder beide Komponenten zusammen zu steuern.

Das erfindungsgemässe Blockcopolymer ist erhältlich durch lineare Polykondensation eines ersten Diols mit einer weiteren Komponente ausgewählt aus der Gruppe eines zweiten Diols, einem α,ω-Dihydroxypolyester oder einem α,ω-Dihydroxypolyether in Anwesenheit von Diisocyanat, Disäurehalogenid oder Phosgen. Durch die Verknüpfung dieser Komponenten werden mit Diisocyanat Polyurethane, mit Disäurehalogenid Polyester und mit Phosgen Polycarbonate erhalten.

Das erste und das zweite Diol (1) können gleich sein oder verschieden und sind erhältlich durch Transesterifikation von α ,ω-Dihydroxy-[oligo(3-(R)-hydroxybutyrat)-ethylenoligo-(3-(R)-hydroxybutyrat)(2), das nachfolgend als PHB-Diol bezeichnet wird, mit Diglycolid oder ε -Captolacton (3), wobei die Transesterifikation bevorzugt in Anwesenheit eines Katalysators durchgeführt wird. Im nachfolgenden Reaktionsschema steht m für 1 bis 50, n für 1 bis 50, x+y für 1 bis 50.

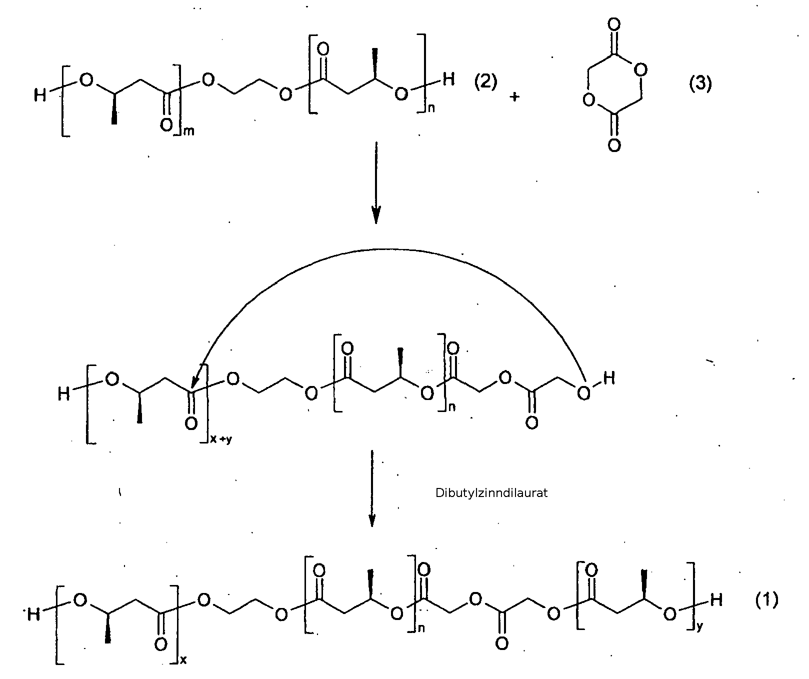

Bevorzuge Katalysatoren sind Transesterfikationskatalysatoren besonders auf der Basis von Zinn, z.B. Dibutylzinndilaurat. Das Diol hat vorzugsweise ein Molekulargewicht von 500 bis 10000 Dalton. Bevorzugt weist das Diol (1) einen totalen Glykolid- oder ε -Captolacton-Gehalt von bis zu 40 mol%, besonders bevorzugt bis zu 30 mol%, auf. Ein bevorzugtes erfindungsgemässes Diol ist α ,ω-Dihydroxy-[oligo(3-R-hydroxybutyrat)-stat-glycolid)-ethylen-oligo-(3R)-hydroxybutyrat-stat-glycolid).

Ein α,ω-Dihydroxypolyester kann beispielsweise durch Transesterifikation von Poly-[(R)-(3)-hydroxy-buttersäure] beziehungsweise deren Copolymeren mit 3-Hydroxyvaleriansäure mit Ethylenglykol erhalten werden.

Als weitere α,ω-Dihydroxypolyester eignen sich Oligomere der α-, β-, γ- und ω-Hydroxycarbonsäuren und deren Cooligomeren, die durch ringöffnende Polymerisation von zyklischen Estern oder Lactonen erhalten werden. Bevorzugte zyklischen Ester dieser Art sind (L,L) Dilactid, (D,D)-Dilactid, (D,L)-Dilactid, Diglycolid oder die bevorzugten Lactone wie β-(R)-Butyrolacton, β -(S)-Butyrolacton, β-rac-Butyrolacton und ε-Caprolacton oder deren Gemische. Die Ringöffnung erfolgt mit aliphatischen Diolen wie Ethylenglykol oder längerkettigen Diolen. Durch die stöchiometrisch eingesetzte Menge dieser Diole wird das Molekulargewicht des erhaltenen Makrodiols bestimmt.

Die ringöffnende Polymerisation der zyklischen Ester oder Lactone erfolgt vorzugsweise in der Masse in Anwesenheit eines Katalysators, beispielsweise SnO(Bu)₂ bei 100°C bis 160°C. Die erhaltenen Makrodiole weisen Molekulargewichte von etwa 300-10'000 Dalton auf. Die aus Gemischen von zyklischen Estern oder Lactonen hergestellten Makrodiole weisen in Abhängigkeit von der Katalysatormenge eine Mikrostruktur auf, die in der Verteilung der monomeren Komponenten zwischen Blockform, statistisch oder alternierend ist. Die Verteilungsstatistik hat einen Einfluss auf die physikalischen Eigenschaften. Beispiele solcher durch ringöffnende Polymerisation von zyklischen Estern und Lactonen in Gegenwart eines Katalysators erhaltene Ester, die zur Herstellung der Blockcopolymere verwendet werden können, sind α ,ω-Dihydroxy-[poly(Llactid)-ethylen-poly(L-lactid)]; α ,ω-Dihydroxy-[oligo(3-(R)-hydroxybutyrat-*ran*-3-(S)-hydroxybutyrat)-ethyleneoligo(3-(R)-hydroxybutyrat- *ran*-3-(S)-hydroxybutyrat)]; α ,ω-Dihydroxy-[oligo(glycolid-*ran*-ε-caprolacton)-ethylenoligo(glycolid-*ran*-ε-caprolacton)]; α,ω-Dihydroxy-[oligo(L)-lactide-*ran*-ε-caprolacton)-ethylen-oligo(L)-lactid-*ran*-ε-caprolacton)]; α,ω-Dihydroxy-[oligo (L)-lactide-*ran*-glycolid)-ethylen-oligo(L)-lactid-*ran*glycolide)]; α,ω- Dihydroxy-[oligo(3-(R)-hydroxybutyrat*ran*-3-(S)-hydroxybutyrat-*ran*-glycolid)-ethylen-oligo(3-(R) hydroxybutyrat-*ran*-3-(S)hydroxybutyrat-*ran*-glycolid); α ,ω-Dihydroxy-[oligo-3-(R)-hydroxybutyrat-*ran*-3-(S)-hydroxybutyrat-*ran*-L-lactid-ethylen-oligo(3-(R)-hydroxybutyrat-*ran*-(S)-hydroxybutyrat-*ran*-L-lactid)] und α ,ω-hydroxy-[oligo(3-(R)-hydroxybutyrat-*ran*-3-(S)-hydroxybutyrat-*ran*-ε-caprolacton) ethylene-oligo (3- (R)-hydroxybutyrat-*ran-*3-(S)-hydroxybutyrat-ran-ε-caprolacton)].

Die ringöffnende Polymerisation zur Herstellung dieser Makrodiole kann auch ohne Katalysator erfolgen. Als Diisocyanate für die Herstellung der Polyurethanvariante der Blockcopolymere eignen sich insbesondere Hexamethylendiisocyanat, 2,2,4-Trimethylhexamethylendiisocyanat, Cyclohexyl-1,4-diisocyanat, Cyclohexyl-1,2-diisocyanat, Isophorondiisocyanat, Methylendicyclohexyldiisocyanat und L-Lysindiisocyanatmethylester.

Für die Herstellung der Polyestervariante der Blockcopolymere eignen sich insbesondere Disäurehalogenide von Oxalsäure, Malonsäure, Bernsteinsäure, Glutarsäure, Adipinsäure, Pimelinsäure, Korksäure, Azelainsäure, Trimethyladipinsäure, Sebazinsäure, Dodecandisäure, Tetradecandisäure und Hexandecandisäure.

Ein besonders bevorzugtes Blockcopolymer ist Poly[poly[α,ω-Dihydroxy-[oligo(3-(R)-hydroxybutyrat)-statglycolid)-ethylen-oligo-(3-(R)-hydroxybutyrat-statglycolid)]alt-2,2,4-trimethylhexamethylen-1,6-diisocyanat]-co-poly[dihydroxy[oligo-glykolid-ran-ε-caprolacton)-ethylen-(oligo-glykolid-ran-ε-caprolacton)]alt-2,2,4-trimethyl hexamethylen-1,6-diisocyanat] der Formel 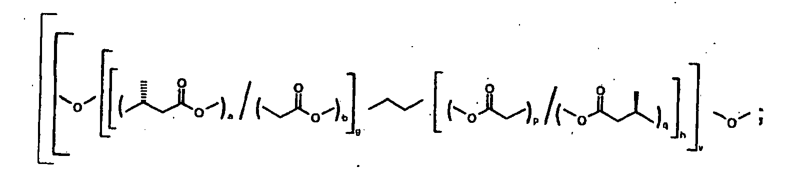 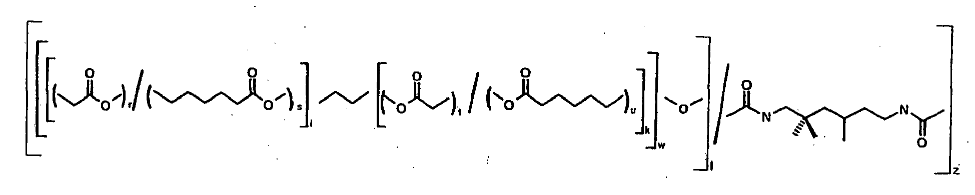 wobei a = 1 bis 50, b = 1 bis 10, p = 1 bis 10, q = 1 bis 50, r = 1 bis 10, s = 1 bis 50, t = 1 bis 10, u = 1 bis 50 und z = 1 bis 50 ist.

Besonders bevorzugt sind Blockcopolymere und Diole, die in fünf bis sechs Tagen im menschlichen oder im tierischen Körper abbaubar sind. Weitere bevorzugte Blockcopolymere und Diole sind solche, deren Abbau über Monate oder Jahre stattfindet. Die Abbaugeschwindigkeit hängt primär von der Anzahl der Diglycolid- oder Glykolideinheiten ab. Bei Lagerung in einer neutralen Pufferlösung bei 37°C nimmt das Molekulargewicht in Abhängigkeit des Glykolidgehalts mit der Zeit ab.

Trotz des relativ hohen Digylkolid- oder Glykolid-Gehalts respektives-Captolacton Gehalts bildet das erfindungsgemässe Blockcopolymer phasensegregierte kristalline Domänen im festen Polymer aus, die die mechanischen Eigenschaften des erfindungsgemässen Blockcopolymers massgeblich bestimmen, wie zum Beispiel die gute Festigkeit, die Sprödigkeit, sowie die erhöhte Bruchdehnung und Bruchspannung.

Die physikalischen Eigenschaften solcher Blockcopolymere werden durch das Massenverhältnis der kristallinen und der amorphen Polymeranteile massgebend gesteuert. Bevorzugt ist dabei ein kristalliner Anteil von 5 bis 50%. Durch das Diol ist die Menge der kristallinen Komponente, die einen massgebendend Einfluss auf die mechanischen Eigenschaften hat, freier wählbar, da die Abbaurate auch durch das Diol gesteuert werden kann.

Die erfindungsgemässen Blockcopolymere und Diole sind ausgesprochen gut löslich in organischen Lösungsmitteln wie Dioxan, chlorierten Lösungsmitteln, DMSO etc. und haben den besonderen Vorteil, dass ihre physikalischen, chemischen und biologischen Eigenschaften durch die Anzahl der Diglycolid-Einheiten respektive ε-Captolacton Einheiten innerhalb eines breiten Spektrums eingestellt werden können. So können die erfindungsgemässen Blockcopolymere und Diole den jeweils spezifischen Verwendungen angepasst werden.

Die Blockcopolymere können durch Copolymerisation mit weiteren niedermolekularen Verbindungen modifiziert werden. Diese copolymerisierten Verbindungen weisen eine oder mehrere funktionellen Gruppen auf. Bei diesen funktionellen Gruppen kann es sich um geschützte oder ungeschützte reaktive Gruppen handeln, oder um Gruppen, die den Diolen bestimmte Verwendungseigenschaften verleihen. Beispielsweise können diese niedermolekularen Verbindungen die Verwendung der Blockcopolymere als Röntgenkontrastmittel oder in anderen diagnostischen Verfahren wie CT und MRI als Mittel zur Kontrasterhöhung ermöglichen. Wenn es sich bei den funktionellen Gruppen um reaktive Gruppen handelt, ermöglichen sie eine kovalente Bindung von Wirkstoffen an das erfindungsgemässe Blockcopolymer. Bei solchen Wirkstoffen handelt es sich beispielsweise um Diagnostika, wie Kontrastmittel, pharmazeutische Wirkstoffe, Peptide, Proteine, etc. Besonders geeignete niedermolekulare Comonomere sind Diatrizoesäure-mono-glycerylester; 10,11-Dihydroxyundecansäure; Phenacyl-10,11-dihydroxyundecanoat; 2,2-Bis-(hydroxymethyl)-propionsäure; Phenacyl-bis-(hydroxymethyl)-propionat. Dem Fachmann ist bekannt, wie solche Wirkstoffe kovalent an das Diol gebunden werden können.

Eine weiter wichtige Eigenschaft des erfindungsgemässen Diols oder der Blockcopolymere sind ihre thermoplastische Verarbeitbarkeit. Im allgemeinen sind sie bei Temperaturen zwischen 80°bis 200°, vorzugsweise zwischen 100°und 150°, verarbeitbar. Die Verarbeitung kann entsprechend nach bekannten Verfahren durch Extrusion und Blasen oder Spritzgiessen erfolgen. Folien sind auch durch Verpressen herstellbar. Diese thermoplastische Verarbeitbarkeit bringt für die medizinischen Implantate den Vorteil der Anpassbarkeit der Form und Grösse des Implantates. Weiterhin kann chirurgisches Nahtmaterial daraus entsprechend verschweisst werden, was den Verzicht auf das komplizierte Verknüpfen ermöglicht. Denkbare Anwendungen ausserhalb des medizinischen Bereichs sind Fasern zur Herstellung von Textilien, Tapeten, Teppichen, Filter und Bautextilien. Diese Fasern sind sowohl Schmelz- als auch Lösungsverspinnbar.

Die Implantate können auch in Form eines Rohres vorliegen. Unter einem Rohr werden auch Schläuche verstanden. Die Rohre können runde, elliptische und mehreckige Querschnitte aufweisen, wobei innerhalb eines Rohres auch mehrere Kanäle angeordnet sein können. Bei den erfindungsgemässen Implantaten kann eine Neubildung einer funktionellen Gefässwand oder eines Nervs erfolgen. Durch einen Überzug mit funktionellen Gefässzellen kann ein thrombotischer Verschluss in der Langzeitanwendung vermieden werden, d.h. das biokompatible Polymer kann durch neue körpereigene Zellen mit der Zeit substituiert werden. Für bestimmte Verwendungen kann das Implantatmaterial eine poröse Struktur aufweisen. Es kann auch Kapselform zur Aufnahme von pharmazeutischen Wirkstoffen oder Diagnostika auch in Form von Partikeln aufweisen.

Nachfolgend werden einige Verwendungen der erfindungsgemässen Diole und der Blockcopolymere im medizinischen Bereich aufgeführt. Selbstverständlich sind weiter Verwendungen möglich.
- Rohrförmige Strukturen (Gefässersatz, Luftröhrenersatz, Ersatz anderer biologischen Rohrstrukturen) in fester, spiralförmiger, flexibler, expandierbarer, selbstexpandierender, geflochtener und gewirkter Form, die entsprechend dem biologischen und funktionellem Bedarf, an der Innen- respektive Aussenseite physikalisch und pharmakologisch adäquat strukturiert oder beschichtet sein können. Die pharmakologischen Substanzen werden entweder durch Absorption oder kovalente chemische Bindung am Diol oder am Blockcopolymer festgehalten. Ebenso eignen sich die Implantatmaterialien zur Herstellung von Stents (starr, expandierbar, selbstexpandierend) für Gefässe oder andere biologische Röhrenstrukturen (Oesophagus, Gallenwege, Harnwege).
- Folienförmige Strukturen (Wundabdeckung, Membranoxygenatoren, Hornhautersatzgrundlage etc.) können ebenfalls mit dem erfindungsgemässen Diol oder dem Blockcopolymer hergestellt werden.
- Fadenförmige Strukturen als chirurgisches Nahtmaterial und zur Verarbeitung zu gewobenen, geflochtenen oder gewirkten Strukturen.
- Clipförmige oder klammerförmige Strukturen für Klammerngeräte oder Klammern zum Unterbinden kleiner Blutgefässe und Ausnützung der thermoplastischen Eigenschaften zum Verschluss.
- Feste bis gelartige oder poröse Strukturen als Matrix für die Herstellung von einfachen oder zusammengesetzten biologischen Geweben in vitro (Tissue engineering in vivo), Anwendung in der topischen Wundbehandlung.
- Prekonditionierte Platzhalter für Hautersatz, Fettgewebe, Sehnen, Knorpel und Knochen, Nerven etc.).
- Polymere Strukturen, die auf Grund der physikalischen respektive biologischen Ladungseigenschaften und physikalischen Strukturen (Schäume, Gel, Mikro- und Nanosphären) und der Oberflächenstruktur, die Abgabe therapeutischer (Hormone, Medikamente) oder kosmetischer (Liposomen, Proteine, Vitaminen) Substanzen über innere anatomische Strukturen oder über Haut ermöglichen.
- Mittel aus dem erfindungsgemässen Material zur Verödung von Varikocelen, Varicen der Beine (Oeosphagusvarizen) oder von gastroindestinalen Blutungsquellen (endoskopisch oder transvaskulär).
- Formkörper, die in geeigneter Form und Beladung mit bioaktiven Substanzen die reversible oder irreversible Antikonzeption durch Blockierung (Ovidukt, Duktus spermaticus) ermöglichen.
- Künstliche Gehörknöchelchen (Ossicles) und künstliche Herzklappen, Aorten und kardiovaskuläre Gefässe.

Das erfindungsgemässe Diol oder Blockcopolymer kann ausserdem als Grundlage für Züchtung von Hornhautzellen auf Folien zur Transplantation als Hornhautersatz verwendet werden. Ausserdem sind weitere Verwendungsmöglichkeiten in entsprechenden physikalischen und oder biologischen Form in den medizinischen Dental-, Mikro- oder Nanotechnologien.

Die erfindungsgemässen Diole sind in *in vitro* Zellkulturen mit Makrophagen und Fibroblasten auf Grund der Beobachtung von Zelladhäsion, Zellwachstum, Zellvitalität und Zellaktivierung sowie der Produktion von extrazellulären Proteinen und Zytokinen äusserst biokompatibel.

Nachfolgend wird die Erfindung anhand von Beispielen weiter veranschaulicht.

### Beispiel 1

Herstellung von α,ω-Dihydroxy [oligo(3-(R)
- hydroxybutyrat)-ethylen-oligo(3-(R)
- hydroxybutyrat)] durch Transesterifizeirung von Poly[(R)-3-hydroxybutyrat] mit Ethylenglykol.

### 1055g Poly[(R)-3-hydroxybutyrat] / Biopol (ICI) werden unter N₂ in 3 1 Diglyme bei 140° C gelöst. Dann werden

246 g Ethylenglykol un 5.21 g Dibutylzinndilaurat (Kat.) zugegeben. Nach einer Stunde wird 1.5 g (125°C) und nach weiteren 2.5 Stunden nochmals 1.2 g Katalysator zugesetzt. Der Abbau wird durch GPC Messungen ständig verfolgt und in Intervallen von 1 h werden zusätzliche 0,6 g Katalysator zugesetzt bis das angestrebte Molekulargewicht des Abbauprodukts erreicht ist. Kontrolle des Molekulargewichtes durch GPC. Der Abbruch des Abbaus erfolgt durch Ausfällen des Polymers in 10 1 Wasser.

Das abgebaute Oligomer wird abfiltriert und insgesamt 5 mal in ca. 6 bis 7 1 dest. Wasser aufgeschlämmt und nach 20 h wieder abfiltriert. Nach dem letzten Waschgang wird das körnige Oligomer während einer Stunde trocken gesaugt und dananch in 2 grosse Kristallisierschalen zuerst im Trockenschank bei 50°C im Vakuum getrocknet. Danach im Hochvakuum (10⁻² bar) für 30 Stunden am Trockenschrank bei 60°C weitergetrocknet.

Die Löslichkeit des getrockneten Oligomers in chlorierten Lösungsmitteln ist sehr ausgeprägt von der Kristallinität des erhaltenen Produkts abhängig.Die Oligomere können beim Lagern Nachkristallisieren. Hochkristalline Oligomere werden in Chloroform heiss gelöst und die heisse Lösung wird durch Soxhlethülsen druckfrei filtriert. Anschliessend werden die abfiltrierten Oligomeren in kaltem Methanol gefällt. Niedriger kristalline Oligomeren werden in warmem Mehylenchlorid gelöst, oder in einem Knofler-Boehm Extraktor heiss mit Methylenchlorid extrahiert so dass eine konzentrierte Lösung resultiert. Das Oligomer wird durch Fällen und Filtrieren aus kaltem Methanol wieder isoliert.

Die Ausbeute des Produkts ist sehr stark von der thermischen Vorgeschichte des Produkts abhängig und beträgt ca 30-50%.

### Reinigung vom Zinnkatalysator:

a)Grobreinigung:Das feste Oligomer wird in einer Soxhlet Apparatur mit Methanol extrahiert. Ca 10% der kelineren Oligomere werden mitextrahiert. Reinheitsbereich bezüglich Zinn um 500 ppm.
b)Das verdünnte Filtrat in Methylenchlorid wird chromatographisch über ein Kieselgel 60µ - Säule gereinigt. Erzielbare Reinheit ca 30 ppm Zinn.

Säulenhöhe ca. 15 cm, Durchmesser 3 cm. Das Filtrat wird aufkonzentriert, bis Oligomere bei 35°C auszufallen beginnen. Dann wird die Lösung (4,-5 1) wurde in 10 1 Petrolether 30/50 gegossen, so dass das Oligomer ausfällt.

Der Niederschlag wird abfiltriert und getrocknet.

### Beispiel 2

### Synthese von α ,ω-Dihydroxy[oligo-3-(R)-hydroxybutyratstat-glycolid)- ethylen-oligo- (3-(R)-hydroxybutyrat-statglycolid)]

Die Transesterifizierung von α,ω -Dihydroxy[oligo-3-(R)-hydroxybutyrat)- ethylen-oligo- (3- (R)-hydroxybutyrat)] mit Diglycolid wurde in einem ölbeheizten doppelwandigen 350 ml Reaktor, der mit einem Temperaturfühler, Kapillare für Stickstoff als Schutzgas und einem Rückflusskühler auf einem Tropftrichter mit Druckausgleich bestückt war, durchgeführt. Der Tropftrichter wurde mit Molekularsieb A4 gefüllt. Als Lösungsmittel diente Diglym, oder Xylol-Isomerengemisch. Das Diol α ,ω-Dihydroxy[oligo-3-(R)-hydroxybutyrat)- ethylen- oligo-(3-(R)-hydroxybutyrat)] wurde in diesem Lösungsmittelgemäss Tabelle 1 gelöst und zum Sieden erhitzt.Die gewünschte Menge des Diglykolids wurde in trockenem Diglym gelöst und mittels einer Dosierpumpe in der gewünschten Menge pro Zeiteinheit langsam zum Reaktorinhalt zugegeben. Der Katalysator Dibutylzinndilaureat wurde zu Beginn der Glykolidzugabe in den Reaktor gegeben. Die Menge des zugegebenen Katalysators lag zwischen 0 - 10 Gew% bezogen auf das Diglykolid. Die gesammte Reaktionsdauer wurde in einigen Versuchen im Vergleich zur Glykolidzugabezeit erhöht, um den Glykolideinbau quantitativer zu bekommen. Die Reaktionstemperatur betrug 140°C, bei E7 130°C und E8 120°C. Nach der Reaktion wurde das Polymer in der 5-fachen Menge n-Hexan gefällt, abfiltriert und getrocknet.

Reinigung von Diydroxy[oligo-3-(R)-hydroxybutyrat-statglycolid)- ethylen-oligo-(3-(R)- hydroxybutyrat-statglycolid)] : Sinkt das Verhältnis bei der Transesterifikation von eingesetzten 3-(R)-hydroxybutyrateinheiten/ Glykolateinheiten unter einen Wert von ca 3, so entsteht gegen das Ende der Transesterifikation im Reaktionsgemisch eine leichte Trübung, die auf die Entstehung unlöslicher Oligoglykolide zurückgeführt werden kann. Das Polymer kann auf folgende Weise von diesen Teilen, dem Katalysator DBTL und von Diglykolid gereinigt werden:
25 g Rohpolymer werden in einem Soxhlet mit Kühlmantel unter Kühlen auf 18°C während 6h mit Methanol extrahiert und danach im Vakuum getrocknet. Anschliessend wird das Polymer im gleichen gekühlten Soxhlet mit trockenem Methylenchlorid extrahiert und mit der fünffachen Menge trockenem Methanol gefällt und am Vakuum getrocknet. Ausbeute: 86% des Rohpolymers.

**Tab. 1 Reaktionsbedingungen**

| Probenbezeichnung | PHB-diolGlykolid [g] | | Zugabemenge [g/h] | Zugabemenge [%/h] | Zugabezeit [h] | Reaktionsdauer [h] | Diglym [ml] |
|---|---|---|---|---|---|---|---|
| E1 | 20.04 | 2.08 | 0.12 | 5.8 | 17.8 | 23.5 | 170 |
| E2 | 20.04 | 2.08 | 0.17 | 8.2 | 12.0 | 12.0 | 170 |
| E3 | 19.73 | 4.2 | 0.35 | 8.3 | 11.0 | 18.0 | 170 |
| E4 | 20.07 | 6.66 | 0.36 | 5.4 | 18.5 | 18.5 | 170 |
| E5 | 20.04 | 6.64 | 0.3 | 4.5 | 22.0 | 22.0 | 170 |
| E6 | 100.02 | 33.75 | 1.02 | 3.0 | 33.0 | 44.0 | 340 |
| E7 | 150.36 | 50.25 | 1.26 | 2.5 | 40.0 | 62.0 | 400 |
| E8 | 20.8 | 5.4 | 0.34 | 6.8 | 16.0 | 33.5 | 200 |

**Tabelle2: Einbau der Glykolateinheiten in die PHB-Diole**

| Probenbezeichnung | Molares Verhältnis der eingesetzten Monomereinheiten HB/G | Molares Verhältnis der im Polymer nach NMR gefundenen Gruppen HB/G | Anteil von transesterifiziertem Glykolid in % | Anteil von transesterifiziertem Glykolid in Blöcken von 3 und mehr Einheiten in % |
|---|---|---|---|---|
| E1 | 6:1 | 34:1 | 18 | 16 |
| E2 | 6:1 | 31:1 | 19 | 16 |
| E3 | 3:1 | 9:1 | 33 | 33 |
| E4 | 2:1 | 3:1 | 67 | 44 |
| E5 | 2:1 | 4.7:1 | 43 | 47 |
| E6 | 2:1 | 4.7:1 | 43 | 33 |
| E7 | 2:1 | 4.7:1 | 27 | 70 |
| E8.5 | 2.5:1 | 4:1 | 63 | 33 |

**Tabelle3: Zeitlicher Verlauf von Experiment 2**

| Probenbezeichnung | Zeit der Probenentnahme nach Reaktions beginn | Zugefügte Glykolidmenge bezogen auf Total [%]* | Maximales Verhältnis 3-(R)-hydroxy-butyrat/Glykolat im Polymer | Gefundenes Verhältnis 3-(R)-hydroxy- butyrat/Glykolat im Polymer | Glykolat-umsatz [%] | Anteil von transeteri fiziertem Glykolid in Blöcken von 3 und mehr Einheiten [%] |
|---|---|---|---|---|---|---|
| E 8.1 | 6.0 | 40 | 6.2:1 | 22:1 | 20 | 20 |
| E 8.2 | 8.5 | 50 | 4.9:1 | 10:1 | 49 | 23 |
| E 8.3 | 14.5 | 88 | 2.8:1 | 5.7:1 | 50 | 33 |
| E 8.4 | 16.0 | 100 | 2.5:1 | 4:1 | 63 | 47 |
| E8.5 | 33.5 | | 2.5:1 | 4:1 | 63 | 33 |

### Beispiel 3

Herstellung von Poly[poly[α,ω -dihydroxy[oligo-3-(R)-hydroxybutyrat-stat-glycolid)- ethylen-oligo-(3-(R)-hydroxybutyrat-stat-glycolid)]-alt-2,2,4-trimethylhexamethylen- 1,6- diisocyanat]-co-poly[α,ω diydroxy[oligo-glykolid-ran-ε-caprolactone)- ethylen-(oligo- glykolid-ran-ε-caprolactone)] -alt-2,2,4-trimethylhexamethylen-1,6-diisocyanat].

Die Polymerisation wurde in einem ölbeheizten doppelwandigen 1000 ml Reaktor, der mit einem Temperaturfühler, Kapillare für Stickstoff als Schutzgas und einem Rückflusskühler auf einem Tropftrichter mit Druckausgleich bestückt war, durchgeführt. Der Tropftrichter wurde mit Molekularsieb A4 gefüllt. Der Reaktor wurde mit 400 ml 1,2- Dichlorethan und 31,3 g Diydroxy[oligo-3-(R)-hydroxybutyrat-stat-glycolid)-ethylen- oligo-(3-(R)-hydroxybutyrat- stat-glycolid)] ,Mₙ = 2440, Produkt aus E7, beschickt und aufgeheizt, bis das Lösungsmittel in den Kühler aufgestiegen war und über das Molekularsieb rückflussierte. Es wurde rückflussiert, bis das Lösungsmittel auf unter 20 ppm getrocknet war. Dann wurden 46,25 g Diydroxy[oligo-glykolid-ran-ε-caprolactone)- ethylen- (oligo-glykolid-ran-ε-caprolactone)] Mₙ= 1320 (3-(R)-hydroxybutyrat/glycolat = 1:1)und 10,01 g 2,2,4- und 1,4,4-Trimethylhexamethylendiisocyanat, Isomerengemisch, zugefügt. Als Katalysator wurden 100 µl Dibutylzinndilaurat zugegeben. Die Polymerisation wurde bei 85°C während 5 Tagen durchgeführt. Während dieser Reaktionsdauer wurde die Reaktion mittels GPC und Infrarotspektroskopie verfolgt. Nach dem dritten Reaktionstag wurden noch weitere 5 Gew.% des amorphen Diols in mehreren Schritten zugesetzt bis das Molekulargewicht unverändert blieb und im IR die Iscyanatbande vollständig verschwunden war. Die Polymerisation wurde durch Ausfällen des Polymers in der fünffachen Menge von kaltem Methanol abgebrochen. Das Polymer wurde abfiltriert und am Vakuum getrocknet.

### Beispiel 4

Hydrolytischer Abbau von Poly[poly[α,ω -dihydroxy[oligo-3-(R)-hydroxybutyrat-stat- glycolid)- ethylen-oligo-(3-(R)-hydroxybutyrat-stat-glycolid)]-alt-2,2,4-trimethylhexamethylen-1,6-diisocyanat]-co-poly[α,ω diydroxy[oligo-glykolid-ran-ε- caprolactone)- ethylen-(oligo-glykolid-ran-ε-caprolactone)] -alt-2,2,4-trimethylhexamethylen-1,6- diisocyanat] im Vergleich zum Referenzpolymer Poly[poly[α,ω-Dihydroxy[oligo-3-(R)-hydroxybutyrat )- ethylen-oligo-(3-(R)-hydroxybutyrat ]-alt-2,2,4- trimethylhexamethylen- 1,6-diisocyanat]-copoly[α,ωdiydroxy[oligo-glykolid-ran-ε- caprolactone)-ethylen- (oligo-glykolid-ran-ε-caprolactone)] -alt-2,2,4-trimethylhexamethylen-1,6- diisocyanat] Glycolid/ε-caprolactone = 1/1 molar ;PHB/glykolid-Diol aus Experiment 1.

Der Einfluss des Glykolid modifizierten PHB-diols auf die Abbaurate wurde bezüglich eines strukturell analogen Polymers mit unmodifiziertem PHB-diol ermittelt. Die Abbauversuche wurden am pulerförmigen Rohpolymer und an Polymerproben durchgeführt, die zuvor zu Filmen und offenporigen Schäumen (Porengrösse ca 50 - 300 µm) verarbeitet wurden.
Vom Polymer aus Beispiel 2 und dem Referenzpolymer wurden je 3 Schaum- und 3 Pulverproben sowie 20 Filmproben angesetzt. Die Einwagen lagen zwischen 0,1 und 1 g. Die Proben wurden in verschliessbaren Plastikgefässen in 40 ml destilliertem Wasser bei 37°C, über einen Zeitraum von bis zu 88 Tagen gelagert. Zur Vermeidung des Algenwachstums wurden 40 mg Natriumazid zu jeder Probe gegeben. Für die Molmassenbestimmung ist in Abständen von einem Tag bis zu drei Wochen abwechselnd aus den drei Kolben, jeweils vom Schaum und Pulver eine kleine Materialmenge entnommen, in Vakuumschrank bei Raumtemperatur getrocknet und die Molmasse mittels GPC bestimmt worden. Für die Zugversuche wurden jeweils 5 Folien entnommen, die im Vakuumschrank bei Raumtemperatur getrocknet wurden, Die Filmproben wurden durch Spannungs- Dehnungsmessungen charakterisiert. Je 5 Filme sowie Schaum und Pulverproben der Ausgangsprodukte sind bei Beginn des Abbauversuches getestet worden.

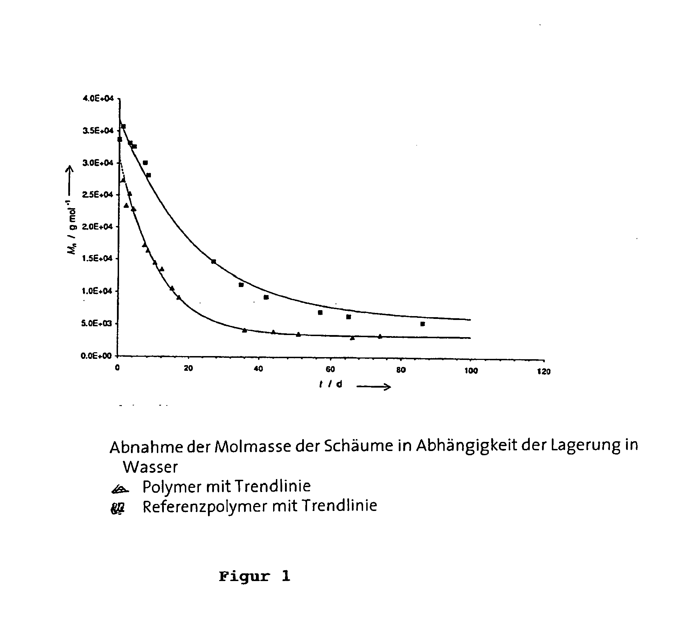

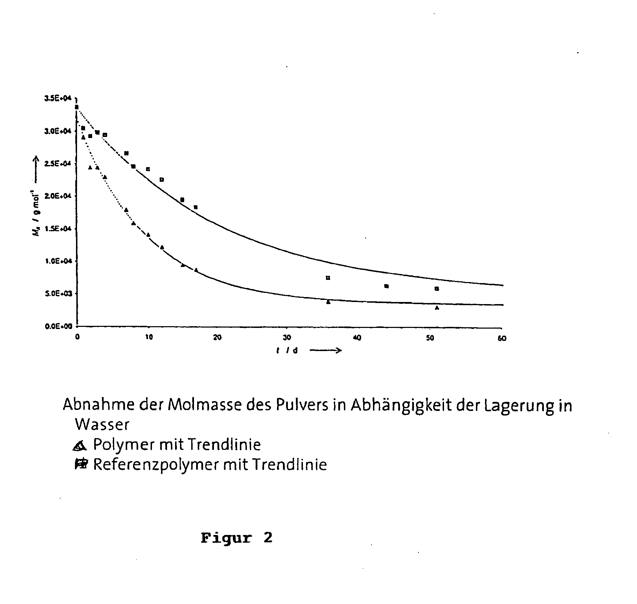

In Figur 1 wird die Abnahme der Molmasse des Schaums gezeigt. In Figur 2 wird die Abnahme der Molmasse des Pulvers gezeigt.
Dabei konnte mit einer Exponentialfunktion mit Trendlinie folgende Halbwertszeit festgestellt werden:

| Probenbezeichnung | Halbwertszeit [d] |
|---|---|
| Polymerschaum | 8.9 |
| Referenzschaum | 19.5 |
| Polymerpulver | 8 |
| Referenzpulver | 18 |

### Beispiel 5

### Synthese von α ,ω-Dihydroxy[oligo-3-(R)-hydroxybutyrat-co-ε-caprolacton)- ethylen-oligo- (3-(R)-hydroxybutyrat-co-ε-caprolacton)]

In einem 101 doppelwandigen Glasreaktor wurden 51 Xylol vorgelegt und anschliessend 4 kg PHB Biomer Pulver dazugegeben und vermischt. Beim Aufheizen des Reaktor auf 140°C begann das Biomer langsam zu schmelzen. Zum schmelzenden Biomer wurden 98.93g Ethylenglykol und 390g - ε-Caprolacton und 100 ml Dibutylzinndilaurat zugegeben. Zur Kontrolle des Abbaus wurden in Intervallen Proben genommen und mittels GPC untersucht. Als nach 22h Reaktionszeit die Molmasse des Abbauprodukts mit einer Referenzsubstanz von 2660 Dalten uübereinstimmte wurde die Reaktion abgebrochen und das Produkt wie unter Beispiel 1 aufgearbeitet.Die NMR Analyse des Produkts zeigte, dass das Produkt die theoretische Menge von ε -Caprolacton enthielt. Ueber die Verteilung der Comonomeren konnte keine Aussage abgeletet werden.
Ausbeute:85%

## Patentansprüche

1. Biokompatibles Blockcopolymer mit mindestens zwei chemisch verschiedenen Blockbausteinen erhältlich durch lineare Polykondensation von einem ersten Diol mit einer Komponente ausgewählt aus der Gruppe eines zweiten Diols, einem α,ω-Dihydroxy-polyester oder einem α,ω-Dihydroxy-polyether in Anwesenheit von Diisocyanat, Disäurehalogenid oder Phosgen,
wobei das erste und das zweite Diol gleich oder verschieden sein können und erhältlich sind durch Transesterifikation von α,ω-Dihydroxy-[oligo(3-(R)-hydroxybutyrat)-ethylen-oligo-3-(R)-hydroxybutyrat) mit Diglycolid oder ε-caprolacton,
der α,ω-Dihydroxy-polyester durch Transesterifikation von Poly-(R)-(3)-hydroxybuttersäure oder deren Copolymeren mit 3-Hydroxyvaleriansäure mit Ethylenglykol erhältlich ist, und
der α,ω-Dihydroxy-polyether ausgewählt ist aus der Gruppe von (α,ω-Dihydroxy-poly(oxytetra-methylen), (α,ω-Dihydroxy-poly(oxyethylen) und Copolymeren von Ethylenglykol und Propylenglykol.

2. Biokompatibles Blockcopolymer nach Anspruch 1, wobei das Blockcopolymer Poly[poly[α,ω-Dihydroxy-[oligo(3-(R)-hydroxybutyrat)-stat-glycolid)-ethylen-oligo-(3-(R)-hydroxybutyrat-stat-glycolid)]alt-2,2,4-trimethylhexamethylen-1,6-diisocyanat]-co-poly[dihydroxy[oligo-glykolid-ran-ε-caprolacton)-ethylen-(oligo-glykolid-ran-ε-caprolacton)]alt-2,2,4-trimethylenhexaethylen-1,6-isocyanat] ist.

3. Biokompatibles Blockcopolymer nach Anspruch 1, wobei das Blockcopolymer Poly[poly[α,ω-Dihydroxy-[oligo(3-(R)-hydroxybutyrat)-co-ε-caprolacton)-ethylen-oligo-(3-(R)-hydroxybutyrat-co-ε-caprolacton)alt-2,2,4-trimethylhexamethylen-1,6-diisocyanat]-co-poly[dihydroxy[oligo-glykolid-ran-ε-caprolacton)-ethylen-(oligo-glykolid-ran-ε-caprolacton)]alt-2,2,4-trimethylenhexaethylen-1,6-isocyanat] ist.

4. Biokompatibles Blockcopolymer nach einem der vorangehenden Ansprüche, **dadurch gekennzeichnet, dass** es biologisch abbaubar ist.

5. Biokompatibles Blockcopolymer nach einem der vorangehenden Ansprüche, **dadurch gekennzeichnet, dass** es im menschlichen und im tierischen Körper abbaubar ist.

6. Biokompatibles Blockcopolymer nach einem der vorangehenden Ansprüche, **dadurch gekennzeichnet, dass** es thermoplastisch verarbeitbar ist.

7. Biokompatibles Blockcopolymer nach einem der vorangehenden Ansprüche, erhältlich durch lineare Cokondensation mit weiteren niedermolekularer Verbindungen mit zusätzlichen funktionellen Gruppen.

8. Biokompatibles Blockcopolymer nach Anspruch 7, **dadurch gekennzeichnet, dass** es chemisch gebundene pharmazeutische Wirkstoffe oder Diagnostika enthält.

9. Formkörper, enthaltend ein biokompatibles Blockcopolymer nach einem der vorangehenden Ansprüche.

10. Medizinisches oder tiermedizinisches Implantat, enthaltend ein biokompatibles Blockcopolymer nach einem der vorangehenden Ansprüche.

11. Implantat nach Anspruch 10, **dadurch gekennzeichnet, dass** es eine poröse Struktur aufweist.

12. Implantat nach einem der Ansprüche 10 oder 11 in Form eines Rohres mit einem oder mehreren Kanälen.

13. Implantat nach einem der Ansprüche 10 oder 12 in Form einer Herzklappe.

14. Chirurgisches Hilfsmittel, bestimmt zur Anbringung im und am menschlichen oder tierischen Körper, enthaltend das biokompatible Blockcopolymer gemäss einem der vorangehenden Ansprüche.

15. Biokompatibles Diol nach Anspruch 1, erhältlich durch Transesterifikation von α,ω-Dihydroxy-[oligo(3-(R)-hydroxybutyrat)-ethylen-oligo-(3R)-hydroxybutyrat) mit Diglycolid.

16. Biokompatibles Diol nach Anpruch 1, erhältlich durch Transesterifikation von α,ω-Dihydroxy-[oligo(3-(R)-hydroxybutyrat)-ethylen-oligo-(3R)-hydroxybutyrat) mit ε-caprolacton.

17. Biokompatibles Diol nach Anspruch 15, wobei das Diol α,ω-Dihydroxy-[oligo(3-R-hydroxybutyrat)-stat-glycolid)-ethylen-oligo-(3R)-hydroxybutyrat-stat-glycolid) ist.

18. Biokompatibles Diol nach Anspruch 16, wobei das Diol α,ω-Dihydroxy-[oligo(3-R-hydroxybutyrat)-co-ε-caprolacton)-ethylen-oligo-(3R)-hydroxybutyrat-co-ε-caprolacton) ist.

19. Verfahren zur Herstellung eines biokompatiblen Diols nach Anspruch 15, **dadurch gekennzeichnet, dass** α,ω-Dihydroxy-[oligo(3-R-hydroxybutyrat)-ethylen-oligo-3-(R)-hydroxybutyrat) mit Diglycolid umgesetzt wird.

20. Verfahren zur Herstellung eines biokompatiblen Diols nach Anspruch 16, **dadurch gekennzeichnet, dass** α,ω-Dihydroxy-[oligo(3-R-hydroxybutyrat)-ethylen-oligo-3-(R)-hydroxybutyrat) mit ε-caprolacton umgesetzt wird.

21. Verfahren nach einem der Ansprüche 19 oder 20, **dadurch gekennzeichnet, dass** die Umsetzung in Anwesenheit eines Katalysators durchgeführt wird.

## Claims

1. A biocompatible block copolymer having at least two chemically different block units obtainable by linear polycondensation of a first diol with a component selected from the group of a second diol, an α,ω-dihydroxypolyester or an α, ω-dihydroxypolyether in the presence of diisocyanate, diacid halide or phosgene,
wherein the first and the second diol may be the same or different, and are obtainable by trans-esterification of α,ω-dihydroxy-[oligo(3-(R)-hydroxybutyrate)-ethylene-oligo-3-(R)-hydroxy-butyrate) with diglycolide or caprolactone,
the α,ω-dihydroxypolyester is obtainable by transesterification of poly-(R)-(3)-hydroxybutyric acid or copolymers thereof with 3-hydroxyvaleric acid with ethylene glycol, and
the α,ω-dihydroxypolyether is selected from the group of α,ω-dihydroxypoly(oxytetramethylene), α,ω-dihydroxypoly(oxyethylene) and copolymers of ethylene glycol and propylene glycol.

2. Biocompatible block copolymer as claimed in claim 1, wherein the block copolymer is poly[poly[α,ω-dihydroxy-[oligo(3-(R)-hydroxybutyrate)-stat-glycolide)-ethylene-oligo-(3-(R)-hydroxybutyrate-stat-glycolide)alt-2,2,4-trimethylhexamethylene 1,6-diisocyanate]-co-poly-[dihydroxy[oligo-glycolide-ran-ε-caprolactone) - ethylene-(oligo-glycolide-ran-ε-caprolactone)]alt-2,2,4-trimethylenehexaethylene 1,6-isocyanate].

3. Biocompatible block copolymer as claimed in claim 1, wherein the block copolymer is poly[poly[α,ω-dihydroxy-[oligo(3-(R)-hydroxybutyrate)-co-ε-caprolacton))]alt-2,2,4-trimethylhexamethylene 1,6-diisocyanate]-co-poly-[dihydroxy[oligo-glycolide-ran-ε-caprolactone)-ethylene-(oligo-glycolide-ran-ε-caprolactone)alt-2,2,4-trimethylenehexaethylene 1,6-isocyanate].

4. Biocompatible block copolymer as claimed in any of the preceding claims, **characterized in that** it is biodegradable.

5. Biocompatible block copolymer as claimed in any of the preceding claims, **characterized in that** it is degradable in the human and in the animal body.

6. Biocompatible block copolymer as claimed in any of the preceding claims, **characterized in that** it is melt-processible.

7. Biocompatible block copolymer to any of the preceding claims, obtainable by linear co-condensation with further low molecular weight compounds having additional functional groups.

8. Biocompatible block copolymer as claimed in claim 7, **characterized in that** it comprises chemically bonded pharmaceutical active substances or diagnostic aids.

9. A shaped article comprising a biocompatible block copolymer as claimed in any of the preceding claims.

10. A medical or veterinary medical implant comprising a biocompatible block copolymer as claimed in any of the preceding claims.

11. An implant as claimed in claim 10, **characterized in that** it has a porous structure.

12. The implant as claimed in either of claims 10 or 11 in the form of a tube having one or more channels.

13. The implant as claimed in either of claims 10 or 12 in the form of a heart valve.

14. A surgical aid intended to be fixed in and on the human or animal body, comprising the biocompatible block copolymer as claimed in any of the preceding claims.

15. Biocompatible Diol according to claim 1, obtainable by transesterification of α,ω-dihydroxy-[oligo(3-(R)-hydroxybutyrate)-ethylene-oligo-(3R)-hydroxybutyrate) with diglycolide.

16. Biocompatible diol according to claim 1, obtainable by transesterification of α,ω-dihydroxy-[oligo(3-(R)-hydroxybutyrate)-ethylene-oligo-(3R)-hydroxybutyrate) with ε-caprolacton.

17. Biocompatible diol according to claim 15, wherein the diol is α,ω-dihydroxy-[oligo(3-R-hydroxybutyrate)-stat-glycolide)-ethylene-oligo-(3R)-hydroxybutyrate-stat-glycolide).

18. Biocompatible diol according to claim 16, wherein the diol is α,ω-dihydroxy-[oligo(3-R-hydroxybutyrate)-co-ε-caprolacton)- -ethylene-oligo-(3R)-hydroxybutyrate- co-ε-caprolacton).

19. A process for preparing a diol as claimed in claim 15, **characterized in that** α,ω-dihydroxy-[oligo(3-R-hydroxybutyrate)-ethylene-oligo-3-(R)-hydroxybutyrate) is reacted with diglycolide.

20. A process for preparing a diol, as claimed in claim 16, **characterized in that** α,ω-dihydroxy-[oligo(3-R-hydroxybutyrate)-ethylene-oligo-3-(R)-hydroxybutyrate) is reacted with ε-caprolacton.

21. The process as claimed in claims 19 or 20, **characterized in that** the reaction is carried out in the presence of a catalyst.

## Revendications

1. Copolymère bloc biocompatible avec au moins deux motifs de bloc chimiquement différents qui est obtenu par polycondensation linéaire d'un premier diol avec un composant choisi parmi le groupe constitué d'un deuxième diol, un α,ω-dihydroxypolyester ou un α,ω-dihydroxypolyéther en présence de diisocyanate, d' halogénure de diacyle ou de phosgène, dans lequel le premier et le deuxième diol peuvent être identiques ou différents et sont obtenus par transestérification de α,ω-dihydroxy-[oligo(3-(R)-hydroxybutyrate)-éthylène-oligo-3- (R)-hydroxybutyrate) avec du diglycolide ou du ε-caprolactone, le α,ω-dihydroxypolyester est obtenu par transestérification d'un acide poly-(R)-(3)-hydroxybutyrique ou de ses copolymères avec l'acide 3-hydroxyvalérique et l'éthylèneglycol, et le α,ω-dihydroxypolyéther est choisi parmi le groupe constitué de α,ω-dihydroxypoly (oxytétraméthylène), α,ω-dihydroxypoly (oxyéthylène) et de copolymères d' éthylèneglycol et propylèneglycol.

2. Copolymère bloc biocompatible selon la revendication 1, dans lequel le copolymère bloc est un poly[poly[α,ω-dihydroxy-[oligo(3-(R)-hydroxybutyrate)-stat-glycolide)-éthylène-oligo-(3-(R)-hydroxybutyrate-stat-glycolide)]alt-2,2,4-triméthylhexaméthyléne-1,6-diiaocyanate]-co-poly[dihydroxy[oligo-glycolide-ran-ε-caprolactone)-éthylène-(oligo-glycolide-ran-ε-caprolactone)]alt-2,2,4-triméthylénehexaéthyléne-1,6-isocyanate].

3. Copolymère bloc biocompatible selon la revendication 1, dans lequel le copolymère bloc est un poly[poly[α,ω-dihydroxy-[oligo(3-(R)-hydroxybutyrate)-co-ε-caprolactone)-éthyléne-oligo-(3-(R)-hydroxybutyrate-co-ε-caprolactone)]alt-2,2,4-triméthylhexaméthylène-1,6-diisccyanate]-co-poly[dihydroxy[oligo-glycolide-ran-ε-caprolactone)-éthylène-(oligo-glycolide-ran-ε-caprolactone)]alt-2,2,4-triméthylènehexaéthyléne-1,6-isocyanate].

4. Copolymère bloc biocompatible selon une des revendications précédentes, **caractérisé en ce qu'**il est biodégradable.

5. Copolymère bloc biocompatible selon une des revendications précédentes, **caractérisé en ce qu'**il est biodégradable dans le corps humain ou animal.

6. Copolymère bloc Biocompatible selon une des revendications précédentes, **caractérisé en ce qu'**il est thermoplastiquement transformable.

7. Copolymère bloc biocompatible selon une des revendications précédentes, obtenu par co-condensation linéaire avec d'autres composées à faible poids moléculaire ayant des groupes fonctionnels supplémentaires.

8. Copolymère bloc biocompatible selon la revendication 7, **caractérisé en ce qu'**il comprend des ingrédients actifs pharmaceutiques ou des agents diagnostiques liés chimiquement.

9. Corps mis en forme, incluant un copolymère bloc biocompatible selon l'une des revendications précédentes.

10. Implant médical ou vétérinaire, incluant un copolymère bloc biocompatible selon l'une des revendications précédentes.

11. Implant selon la revendication 10, **caractérisé en ce qu'**il présente une structure poreuse.

12. Implant selon l'une des revendications 10 ou 11 sous la forme d'un tube avec un ou plusieurs canaux.

13. Implant selon l'une des revendications 10 ou 12 sous la forme d'une valve cardiaque.

14. Auxiliaire chirurgical, destiné à être appliqué dans et sur le corps humain ou animal, incluant un copolymère bloc biocompatible selon l'une des revendications précédentes.

15. Diol biocompatible selon la revendication 1, obtenu par transestérification de α,ω-dihydroxy-[oligo(3-(R)-hydroxybutyrate)-éthylène-oligo-(3R)-hydroxybutyrate) avec du diglycolide.

16. Diol Biocompatible selon la revendication 1, obtenu par transestérification de α,ω-dihydroxy-[oligo(3-R-hydroxybutyrate)-éthylène-oligo-(3R)-hydroxybutyrate) avec du ε-caprolactone.

17. Diol biocompatible selon la revendication 15, dans lequel le diol est du α,ω-dihydroxy-[oligo(3-R-hydxoxybutyrate)-stat-glycolide)-éthyléne-oligo-(3R)-hydroxybutyrate-stat-glycolide).

18. Diol biocompatible selon la revendication 16, dans lequel le diol est du α,ω-dihydroxy-[oligo(3-R-hydroxybutyrate)-co-ε-caprolactone)-éthylène-oligo-(3R)-hydroxybutyrate-co-ε-caprolactone).

19. Procédé pour la production d'un diol biocompatible selon la revendication 15, **caractérisé en ce que** le α,ω-dihydroxy-[oligo(3-R-hydroxybutyrate)-éthylène-oligo-3-(R)-hydroxybutyrate) est amené à réagir avec du diglycolide.

20. Procédé pour la production d'un diol biocompatible selon la revendication 16, **caractérisé en ce que** le α,ω-dihydroxy-[oligo(3-R-hydroxybutyrate)-éthylène-oligo-3-(R)-hydroxybutyrate) est amené à réagir avec du δ-caprolactone.

21. Procédé selon l'une des revendications 19 ou 20, **caractérisé en ce que** la réaction est effectuée en présence d'un catalyseur.
